# EUROPÄISCHE PATENTSCHRIFT

(11) **EP 3 253 272 B1**
(45) Veröffentlichungstag und Bekanntmachung des Hinweises auf die Patenterteilung: **23.09.2020**
(21) Anmeldenummer: 16700835.8
(22) Anmeldetag: 18.01.2016
(51) Int. Cl.: A61B 1/00, G02B 25/00, G02B 23/24

(54) **OKULAREINRICHTUNG UND CHIRURGISCHES INSTRUMENT MIT EINER OKULAREINRICHTUNG**
OCULAR DEVICE AND SURGICAL INSTRUMENT HAVING AN OCULAR DEVICE
DISPOSITIF OCULAIRE ET INSTRUMENT CHIRURGICAL COMPRENANT UN DISPOSITIF OCULAIRE

(30) Priorität: 05.02.2015 DE 102015202002
(43) Veröffentlichungstag der Anmeldung: 13.12.2017
(73) Patentinhaber: Olympus Winter & Ibe GmbH, 22045 Hamburg (DE)
(72) Erfinder: KIEDROWSKI, Gregor, 22397 Hamburg (DE)
(74) Vertreter: Seemann & Partner Patentanwälte mbB
(86) Internationale Anmeldenummer: PCT/EP2016/050866
(87) Internationale Veröffentlichungsnummer: WO 2016/124379

(56) Entgegenhaltungen:
- EP-A2- 2 674 096
- DE-A1- 3 708 124
- DE-A1- 19 507 205
- JP-A- H07 199 090
- JP-A- S58 184 922
- JP-B2- 2 993 281
- US-A- 5 569 163
- US-A1- 2013 267 783

## Beschreibung

Die Erfindung betrifft eine Okulareinrichtung für ein chirurgisches Instrument, insbesondere ein Endoskop, mit einer optischen Baugruppe, die in einer Okularfensterfassung in einem Innenraum des chirurgischen Instruments aufnehmbar oder aufgenommen ist, wobei die optische Baugruppe durch ein Okularfenster von einem Außenraum getrennt ist und das Okularfenster in einer Halterung aufgenommen ist, die mit der Okularfensterfassung verbindbar oder verbunden ist, wobei im verbundenen Zustand die Halterung in einer Klemmrichtung eine Haltekraft auf das Okularfenster ausübt. Ferner betrifft die Erfindung ein chirurgisches Instrument, insbesondere ein Endoskop mit einer Okulareinrichtung.

Endoskope für minimalinvasive Eingriffe am menschlichen oder tierischen Körper sind allgemein bekannt. Mithilfe einer Optik an der distalen Spitze des Endoskopschafts wird die Möglichkeit geschaffen, ein Operations- oder Untersuchungsfeld im Körperinneren zu betrachten. Hierzu sind im Endoskopschaft mehrere optische Baugruppen angeordnet, mit denen Licht aus einem Körperhohlraum hinaus zu einem proximalen Ende des Endoskops geführt wird, an dem das Endoskop von einem Operateur gehalten und bedient wird.

Am proximalen Ende des Endoskops, beispielsweise an einem Griff, befindet sich vielfach ein Okulartrichter mit einem Okular, also einer optischen Baugruppe, aus der das an der distalen Spitze des Endoskops eingetretene Licht wieder austritt. Ein solches Okular kann zur direkten Beobachtung des Operationsfeldes mit bloßem Auge verwendet werden. Vielfach ist vorgesehen, dass an das Okular ein Kamerakopf angeschlossen ist, so dass das Operationsfeld auf einem Monitor betrachtet oder die erfassten Bilddaten einer sich anschließenden Bildverarbeitung zugeführt werden können. Ein solches Endoskop geht beispielsweise aus EP 0 501 088 A1 hervor.

Die in einem Innenraum des Endoskops, beispielsweise in dessen Schaft, vorhandenen optischen Baugruppen sind gegenüber einem Außenraum, der das Endoskop umgibt, durch ein Okularfenster voneinander getrennt.

Aus der EP 2 674 096 A2 ist ein Endoskop bekannt, an dessen proximalem Ende sich ein Eintrittsfenster befindet. Dieses ist schwimmend zwischen zwei O-Ringdichtungen gelagert. Die äußere Dichtung wird über eine Federscheibe, die sich an der Okularmuschel abstützt, in distaler Richtung verspannt. Auf der distalen Seite wird das Eintrittsfenster durch eine Dichtung gehalten, die sich auf einer Federhülse abstützt.

Es ist eine Aufgabe der Erfindung, eine Okulareinrichtung und ein chirurgisches Instrument mit einer Okulareinrichtung anzugeben, wobei die Gefahr für eine Verunreinigung der optischen Baugruppe verringert und die Hygiene verbessert werden soll.

Die Aufgabe wird gelöst durch eine Okulareinrichtung für ein chirurgisches Instrument, insbesondere ein Endoskop, mit einer optischen Baugruppe, die in einer Okularfensterfassung in einem Innenraum des chirurgischen Instruments aufnehmbar oder aufgenommen ist, wobei die optische Baugruppe durch ein Okularfenster von einem Außenraum getrennt ist und das Okularfenster in einer Halterung aufgenommen ist, die mit der Okularfensterfassung verbindbar oder verbunden ist, wobei im verbundenen Zustand die Halterung in einer Klemmrichtung eine Klemmkraft auf das Okularfenster ausübt, wobei zumindest ein die Klemmkraft übertragendes elastisches Element umfasst ist und wobei die Halterung einen mit der Okularfensterfassung verbindbaren oder verbundenen Rand und einen sich daran anschließenden Kragen umfasst, wobei der Kragen eine Öffnung umgibt, wobei die Okulareinrichtung dadurch fortgebildet ist, dass in der Öffnung das Okularfenster zumindest teilweise aufgenommen ist und der Kragen an seiner dem Okularfenster zugewandten Unterseite einen ersten Klemmbereich umfasst, wobei das Okularfenster eine entlang seines Außenumfangs verlaufende Aussparung aufweist, wobei das Okularfenster im Bereich der Aussparung an einer dem Kragen zugewandten Oberseite einen zweiten Klemmbereich aufweist, wobei das elastische Element zwischen dem ersten und dem zweiten Klemmbereich angeordnet ist, und wobei zwischen dem ersten und zweiten Klemmbereich ein Klemmspalt vorhanden ist und das elastische Element in dem Klemmspalt angeordnet ist.

Die erfindungsgemäße technische Lehre beruht auf der folgenden Erkenntnis: Bei herkömmlichen Okulareinrichtungen chirurgischer Instrumente, beispielsweise bei Okulareinrichtungen von Endoskopen, verbleibt vielfach ein Spalt zwischen der Halterung und der Okularfensterfassung, der dem Toleranzausgleich beim Einsetzen des Okularfensters dient. In diesem Bereich des chirurgischen Instruments besteht jedoch die Gefahr, dass sich Schmutzpartikel ansammeln, die nur mit hohem Aufwand entfernt werden können.

Ferner besteht die Möglichkeit, dass zwischen der Halterung und dem Okularfenster Schmutz oder Staubpartikel in den Innenraum des chirurgischen Instruments eindringen und die optische Qualität der optischen Baugruppe verringern. Zur Reinigung der optischen Baugruppe ist es notwendig, das Okularfenster zu entfernen, was mit erheblichem Aufwand verbunden ist. Bei der Okulareinrichtung gemäß Aspekten der Erfindung ist vorteilhaft ein elastisches Element vorgesehen, welches die Klemmkraft überträgt. Der stets notwendige Toleranzausgleich findet somit über eine mehr oder weniger starke bevorzugt elastische Verformung dieses Elements statt. So können Spalte, die ansonsten durch den Toleranzausgleich bedingt auftreten, weitestgehend vermieden werden.

Ferner wird vorteilhaft vermieden, dass beim Anziehen der Halterung das Okularfenster unter starke Spannung gerät. Das elastische Element wirkt in diesem Zusammenhang als Dämpfung.

Gemäß einer Ausführungsform ist die Okulareinrichtung dadurch fortgebildet, dass das elastische Element in der Klemmrichtung betrachtet zumindest abschnittsweise zwischen der Halterung und dem Okularfenster angeordnet ist.

Vorteilhaft wird so eine Abdichtung zwischen dem Okularfenster und der Halterung geschaffen, so dass Schmutz- oder Staubpartikel praktisch nicht mehr in die Okulareinrichtung eindringen können.

Bevorzugt ist die Okulareinrichtung ferner dadurch fortgebildet, dass das elastische Element ein Federelement ist, wobei das Federelement insbesondere eine Tellerfeder, ein Federring und/oder eine Wellenscheibe ist. Ebenso geeignet sind zum Beispiel gummielastische Materialien, beispielsweise in der Form eines O-Rings.

Gemäß einer Ausführungsform ist die Okulareinrichtung dadurch fortgebildet, dass die Okularfensterfassung einen Anschlag für die Halterung umfasst, der eine Bewegung der Halterung in Klemmrichtung begrenzt.

Vorteilhaft wird die Möglichkeit geschaffen, während der Montage das Okularfenster durch das elastische Element spiellos aufzunehmen und anschließend die Halterung anzuziehen, bis diese an den Anschlag anschlägt. Ein Spalt zwischen der Okularfensterfassung, welche den Anschlag umfasst, und der Halterung wird vorteilhaft vermieden. Dies verbessert die Hygieneeigenschaften der Okulareinrichtung bzw. des chirurgischen Instruments, in dem die Okulareinrichtung verbaut wird.

Die Okulareinrichtung ist ferner dadurch fortgebildet, dass der Anschlag eine Bewegung der Halterung in Klemmrichtung derart begrenzt, dass im angezogenen Zustand der Halterung, wenn die Halterung an den Anschlag anstößt, in der Klemmrichtung betrachtet zwischen der Halterung und dem Okularfenster ein Klemmspalt vorhanden ist, wobei das elastische Element zumindest abschnittsweise in dem Klemmspalt angeordnet ist.

Indem ein Klemmspalt zwischen der Halterung und dem Okularfenster konstruktiv vorgesehen wird, wird vermieden, dass das Okularfenster unter zu starke Spannung gerät, auch wenn die Halterung bis zum Anschlag angezogen wird. Eine Überlastung des Okularfensters, welche möglicherweise sogar mit einer Beschädigung des Okularfensters einhergeht, wird vorteilhaft vermieden.

Insbesondere ist die Okulareinrichtung dadurch fortgebildet, dass das Federelement eine an der Halterung, insbesondere an einer Unterseite des Kragens der Halterung, einseitig angeschlagene Tellerfeder umfasst, wobei insbesondere vorgesehen ist, dass sich die Tellerfeder ausgehend von einem Anschlagbereich in Richtung ihres freien Endes von einer Außenseite der Halterung in Richtung eines Zentrums der Halterung erstreckt.

Durch die vorgesehene Ausrichtung bzw. Anordnung der Tellerfeder wird das Eindringen von Schmutz- oder Staubpartikeln in einen Bereich zwischen Okularfenster und Halterung und von dort weiter in Richtung der optischen Baugruppe weitestgehend vermieden.

Gemäß einer weiteren Ausführungsform ist ferner vorgesehen, dass der Kragen zumindest abschnittsweise als Federelement ausgebildet ist. Wird also ein Teil der Halterung, insbesondere deren Kragen, als Federelement ausgebildet, kann vorteilhaft auf ein Bauteil, nämlich das elastische Element selbst, verzichtet werden. Dieses ist nämlich von dem Kragen der Halterung selbst gebildet. Diese Maßnahme vereinfacht die Herstellung der Okulareinrichtung und bietet darüber hinaus Kostenvorteile.

Ferner ist insbesondere vorgesehen, dass die Halterung eine Überwurfmutter ist, wobei insbesondere vorgesehen ist, dass die Überwurfmutter ein Innengewinde aufweist, welches im montierten Zustand mit einem an der Okularfensterfassung vorhandenen Außengewinde kämmt.

Die Halterung und/oder die Okularfensterfassung sind bevorzugt aus einem Metall oder einem Kunststoff hergestellt. Das elastische Element ist ebenfalls beispielsweise aus einem Metall, beispielsweise aus Federstahl, oder aus einem Kunststoff hergestellt. Ebenfalls geeignet sind beispielsweise Gummi oder ähnliche geeignete elastische Materialien. Der Klemmspalt ist ferner bevorzugt zwischen dem ersten und dem zweiten Klemmbereich vorhanden. Das elastische Element ist insbesondere in dem Klemmspalt vorhanden bzw. angeordnet. Beispielsweise handelt es sich bei dem elastischen Element um einen O-Ring oder dergleichen. Das Okularfenster ist bevorzugt als Planglas ausgebildet, weist also zwei planparallele Oberflächen auf. Ferner bevorzugt ist das Okularfenster kreisrund. Gleiches gilt für die Okularfensterfassung. Ein Seitenrand des Okularfensters und die Okularfensterfassung sind ferner bevorzugt form- und funktionskomplementär aufgebaut. Zu diesem Zweck weist das Okularfenster beispielsweise an seinem äußeren Rand eine Aussparung auf, in die die Halterung eingreift. Dieser Seitenrand des Okularfensters ist bevorzugt ringförmig ausgebildet. Erfindungsgemäss sind die Okularfensterfassung und das Okularfenster miteinander verklebt. Beispielsweise ist das Okularfenster entlang seines Umfangs, insbesondere vollständig entlang seines Umfangs, an seinem äußeren Rand mit der Okularfensterfassung verklebt. Bei dem chirurgischen Instrument handelt es sich ferner bevorzugt um ein Laparoskop. Die zuvor genannten Aspekte betreffen vorteilhaft alle Ausführungsformen.

Weitere Merkmale der Erfindung werden aus der Beschreibung erfindungsgemäßer Ausführungsformen zusammen mit den Ansprüchen und den beigefügten Zeichnungen ersichtlich. Erfindungsgemäße Ausführungsformen können einzelne Merkmale oder eine Kombination mehrerer Merkmale erfüllen.

Die Erfindung wird nachstehend ohne Beschränkung des allgemeinen Erfindungsgedankens anhand von Ausführungsbeispielen unter Bezugnahme auf die Zeichnungen beschrieben, wobei bezüglich aller im Text nicht näher erläuterten erfindungsgemäßen Einzelheiten ausdrücklich auf die Zeichnungen verwiesen wird. Es zeigen:
- Fig. 1: eine schematische und vereinfachte Seitenansicht eines chirurgischen Instruments,
- Fig. 2: eine Detailansicht einer Okulareinrichtung in einem schematischen und vereinfachten Längsschnitt und
- Fig. 3: eine schematische und vereinfachte Detailansicht des aus Fig. 2 bekannten Längsschnitts durch die Okulareinrichtung.

In den Zeichnungen sind jeweils gleiche oder gleichartige Elemente und/oder Teile mit denselben Bezugsziffern versehen, so dass von einer erneuten Vorstellung jeweils abgesehen wird.

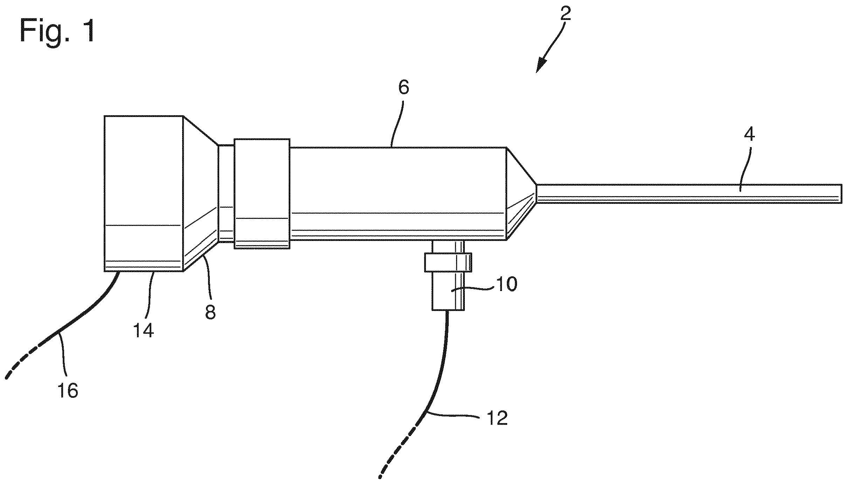

Fig. 1 zeigt in schematischer und vereinfachter Seitenansicht ein chirurgisches Instrument 2, beispielhaft ein Endoskop. An seinem distalen Ende umfasst dieses einen rohrförmigen Schaft 4 mit einer Optik, welche es erlaubt, einen Operations- oder Untersuchungsbereich, welcher distal vor dem freien Ende des Schafts 4 liegt, zu beobachten. Der Schaft 4 mündet in ein Gehäuse 6, das am proximalen Ende einen Okulartrichter 8 aufweist. Das Gehäuse 6 dient der Handhabung des chirurgischen Instruments 2. Seitlich an dem Gehäuse 6 befindet sich eine Lichtquelle 10, beispielsweise eine LED-Lichtquelle. Diese ist über ein Anschlusskabel 12 mit einer geeigneten Stromversorgung verbunden.

Am Okulartrichter 8 ist, schematisch dargestellt, ein Kamerakopf 14 mit einem nicht dargestellten Okularadapter angeordnet. Der Kamerakopf 14 erfasst das aus dem Okular des chirurgischen Instruments 2 austretende Licht mit einer eigenen Optik und bildet dieses auf einem optischen Flächensensor, beispielweise einem CCD- oder CMOS-Chip ab. Mittels eines Anschlusses 16 wird der Kamerakopf 14 mit Strom versorgt. Ferner ist es über den Anschluss 16 möglich, Bildsignale von dem Flächensensor des Kamerakopfs 14 einer externen Auswerteeinheit zu übermitteln und Steuersignale an den Kamerakopf 14 zu übertragen.

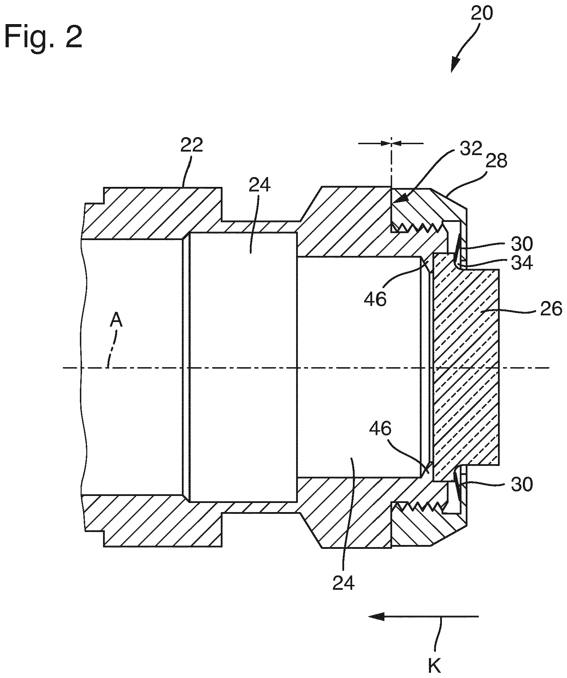

Fig. 2 zeigt in einem schematischen und vereinfachten Längsschnitt eine Detailansicht einer Okulareinrichtung 20, wie sie im Bereich des Okulartrichters 8 am proximalen Ende eines chirurgischen Instruments 2, beispielsweise eines Endoskops, vorgesehen ist.

Die Okulareinrichtung 20 umfasst eine Okularfensterfassung 22, in der eine optische Baugruppe 24 aufgenommen ist. Bei der optischen Baugruppe 24 handelt es sich beispielsweise um eine oder mehrere Linsen oder Linsengruppen, Prismen, Filter oder dgl., welche ein optisches System bilden. Die optische Achse A des optischen Systems ist in strichpunktierter Linie dargestellt. Die Okulareinrichtung 20 befindet sich in einem Innenraum des chirurgischen Instruments 2, beispielsweise in einem von dem Gehäuse 6 des in Fig. 1 dargestellten Endoskops umschlossenen Innenraum. Das von Ihr aufgenommene optische System dient der Abbildung von am distalen Ende in den Schaft 6 aus einem Untersuchungsbereich eintretenden Lichtstrahl auf einen flächigen Sensor des Kamerakopfs 14.

Der Kamerakopf 14 ist abnehmbar. In diesem Zustand trennt ein von der Okulareinrichtung umfasstes Okularfenster 26 den Außenraum vom Innenraum des chirurgischen Instruments 2.

Das Okularfenster 26 ist in einer Halterung 28 aufgenommen. Lediglich beispielhaft ist in Fig. 2 als Halterung 28 eine Überwurfmutter dargestellt. Diese weist ein Innengewinde auf, welches mit einem an der Okularfensterfassung 22 vorhandenen Außengewinde kämmt. Die Halterung 28 ist auf diese Weise mit der Okularfensterfassung 22 verbindbar oder verbunden. Im verbundenen Zustand, den Fig. 2 zeigt, übt die Halterung 28 in einer Klemmrichtung K eine Klemmkraft auf das Okularfenster 26 aus.

In Klemmrichtung K betrachtet befindet sich zwischen der Halterung 28 und dem Okularfenster 26 zumindest ein die Klemmkraft übertragendes elastisches Element 30. Beispielhaft handelt es sich bei dem elastischen Element um eine Tellerfeder. Ebenso ist es jedoch vorgesehen, als elastisches Element einen Federring, eine Wellscheibe, einen O-Ring oder dergleichen zu verwenden.

Ferner umfasst die Okularfensterfassung 22 einen Anschlag 32, der die Bewegung der Halterung 28 in Klemmrichtung K begrenzt. Der Anschlag 32 begrenzt die Bewegung der Halterung 28 in Klemmrichtung K derart, dass im angezogenen Zustand der Halterung 28, wie in Fig. 2 gezeigt, die Halterung 28 an den Anschlag 32 anstößt. Zwischen dem Okularfenster 26 und der Halterung 28 verbleibt jedoch ein Klemmspalt 34. In dem Klemmspalt 34 ist zumindest abschnittsweise das elastische Element 30 angeordnet.

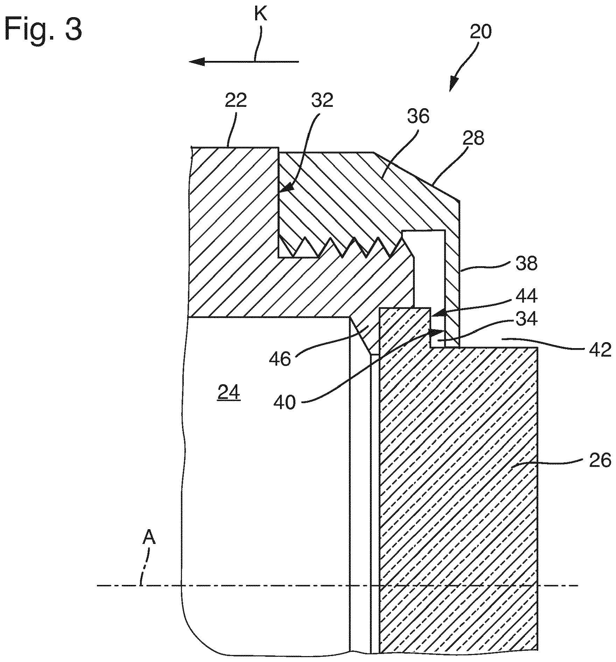

Fig. 3 zeigt in schematischer vereinfachter Längsschnittansicht ein Detail der aus Fig. 2 bekannten Okulareinrichtung 20. Die Halterung 28 ist bevorzugt so ausgestaltet, dass sie einen mit der Okularfensterfassung 22 verbindbaren Rand 36 und einen sich daran anschließenden Kragen 38 umfasst. Der Rand 36 ist auf seiner der Okularfensterfassung 22 zugewandten Innenseite mit einem Innengewinde versehen. In Richtung der optischen Achse A schließt sich an den Rand 36 der Kragen 38 an, der eine Öffnung umgibt, durch die das Okularfenster 26 teilweise hindurchtritt. Mit anderen Worten ist in der von dem Kragen 38 umgebenen Öffnung das Okularfenster 26 zumindest teilweise aufgenommen.

Der Kragen 38 weist an seiner dem Okularfenster 26 zugewandten Unterseite einen ersten Klemmbereich 40 auf. Das Okularfenster 26 weist eine entlang seines Außenumfangs verlaufende Aussparung 42 auf. In diesem Bereich weist das Okularfenster 26 auf seiner dem Kragen 38 zugewandten Oberseite einen zweiten Klemmbereich 44 auf. Das elastische Element 30, welches in Fig. 3 aus Gründen der Übersichtlichkeit nicht dargestellt ist, befindet sich zwischen dem ersten Klemmbereich 40 und dem zweiten Klemmbereich 44.

Die Halterung 28 ist so dimensioniert, dass sie an dem Anschlag 32 der Okularfensterfassung 22 anschlägt und dennoch ein Klemmspalt 34 vorhanden bleibt. So wird vorteilhaft vermieden, dass ein zu großer Druck auf das Okularfenster 26 ausgeübt wird. Gleichzeitig ist sichergestellt, dass zwischen der Halterung 28 und der Okularfensterfassung 22, dort wo sich der Anschlag 32 befindet, kein Spalt an der Außenseite entsteht, in welchem sich ggf. Schmutzpartikel ansammeln könnten.

Ferner wird vorteilhaft das Eindringen von Schmutzpartikeln oder Staub in den Innenraum der Okularfensterfassung 22 vermieden. Durch das elastische Element 30 wird der Spalt zwischen Halterung 28 und Okularfenster 26 dicht verschlossen. Hierzu ist es besonders vorteilhaft, wenn das elastische Element 30, bei dem es sich beispielsweise um ein Federelement handelt, an der Unterseite des Kragens 38 einseitig angeschlagen ist. Die als elastisches Element 30 vorgesehene Tellerfeder erstreckt sich ausgehend von einem Anschlagbereich in Richtung ihres freien Endes von einer Außenseite der Halterung 28 in Richtung deren Zentrum, also in Richtung der optischen Achse A. Somit liegt, wie in Fig. 2 dargestellt, der Kontaktpunkt zwischen der Tellerfeder und dem Okularfenster 26 im Bereich eines Kniepunkts der Aussparung 42. Dies verhindert das Eintreten von Schmutz- oder Staubpartikeln.

Die Okularfensterfassung 22 weist eine Schulter 46 auf, die sich entlang des von der Okularfensterfassung 22 umschlossenen Innenraums, insbesondere vollständig, entlang des Umfangs erstreckt. An einer dem Okularfenster 26 zugewandten ringförmigen Fläche der Schulter 46 ist das Okularfenster 26 mit der Okularfensterfassung 22 verklebt.

Gemäß einem weiteren Ausführungsbeispiel ist vorgesehen, dass der Kragen 38 der Halterung 28 selbst zumindest abschnittsweise als Federelement ausgebildet ist und somit als elastisches Element 30 wirkt.

Alle genannten Merkmale, auch die den Zeichnungen allein zu entnehmenden sowie auch einzelne Merkmale, die in Kombination mit anderen Merkmalen offenbart sind, werden allein und in Kombination als erfindungswesentlich angesehen. Erfindungsgemäße Ausführungsformen können durch einzelne Merkmale oder eine Kombination mehrerer Merkmale erfüllt sein. Im Rahmen der Erfindung sind Merkmale, die mit "insbesondere" oder "vorzugsweise" gekennzeichnet sind, als fakultative Merkmale zu verstehen.

### Bezugszeichenliste

- 2: chirurgisches Instrument
- 4: Schaft
- 6: Gehäuse
- 8: Okulartrichter
- 10: Lichtquelle
- 12: Anschlusskabel
- 14: Kamerakopf
- 16: Anschluss
- 20: Okulareinrichtung
- 22: Okularfensterfassung
- 24: optische Baugruppe
- 26: Okularfenster
- 28: Halterung
- 30: elastisches Element
- 32: Anschlag
- 34: Klemmspalt
- 36: Rand
- 38: Kragen
- 40: erster Klemmbereich
- 42: Aussparung
- 44: zweiter Klemmbereich
- 46: Schulter

- A: optische Achse
- K: Klemmrichtung

## Patentansprüche

1. Okulareinrichtung (20) für ein chirurgisches Instrument (2), insbesondere ein Endoskop, mit einer optischen Baugruppe (24), die in einer Okularfensterfassung (22) in einem Innenraum des chirurgischen Instruments (2) aufnehmbar oder aufgenommen ist, wobei die optische Baugruppe (24) durch ein Okularfenster (26) von einem Außenraum getrennt ist und das Okularfenster (26) in einer Halterung (28) aufgenommen ist, die mit der Okularfensterfassung (22) verbindbar oder verbunden ist, wobei im verbundenen Zustand die Halterung (28) in einer Klemmrichtung (K) eine Klemmkraft auf das Okularfenster (26) ausübt, wobei zumindest ein die Klemmkraft übertragendes elastisches Element (30) umfasst ist, und wobei die Halterung (28) einen mit der Okularfensterfassung (22) verbindbaren oder verbundenen Rand (36) und einen sich daran anschließenden Kragen (38) umfasst, wobei der Kragen (38) eine Öffnung umgibt, in der das Okularfenster (26) zumindest teilweise aufgenommen ist und der Kragen (38) an seiner dem Okularfenster (26) zugewandten Unterseite einen ersten Klemmbereich (40) umfasst, wobei das Okularfenster (26) eine entlang seines Außenumfangs verlaufende Aussparung (42) aufweist, wobei das Okularfenster (26) im Bereich der Aussparung (42) an einer dem Kragen (38) zugewandten Oberseite einen zweiten Klemmbereich (44) aufweist, wobei das elastische Element (30) zwischen dem ersten und dem zweiten Klemmbereich (40, 44) angeordnet ist, und wobei zwischen dem ersten und zweiten Klemmbereich (40, 44) ein Klemmspalt vorhanden ist und das elastische Element (30) in dem Klemmspalt angeordnet ist und wobei
die Okularfensterfassung (22) eine Schulter (46) aufweist, die sich entlang eines von der Okularfensterfassung (22) umschlossenen Innenraums entlang des Umfangs erstreckt, **dadurch gekennzeichnet dass** das Okularfenster (26) an einer dem Okularfenster (26) zugewandten ringförmigen Fläche der Schulter (46) mit der Okularfensterfassung (22) verklebt ist.

2. Okulareinrichtung (20) nach Anspruch 1, **dadurch gekennzeichnet, dass** das elastische Element (30) in der Klemmrichtung (K) betrachtet zumindest abschnittsweise zwischen der Halterung (28) und dem Okularfenster (26) angeordnet ist.

3. Okulareinrichtung (20) nach Anspruch 1 oder 2, **dadurch gekennzeichnet, dass** das elastische Element (30) ein Federelement ist, wobei das Federelement insbesondere eine Tellerfeder, ein Federring und/oder eine Wellenscheibe ist.

4. Okulareinrichtung (20) nach einem der Ansprüche 1 bis 3, **dadurch gekennzeichnet, dass** die Okularfensterfassung (22) einen Anschlag (32) für die Halterung (28) umfasst, der eine Bewegung der Halterung (28) in Klemmrichtung (K) begrenzt.

5. Okulareinrichtung (20) nach Anspruch 4, **dadurch gekennzeichnet, dass** der Anschlag (32) eine Bewegung der Halterung (28) in Klemmrichtung (K) derart begrenzt, dass im angezogenen Zustand der Halterung (28), wenn die Halterung (28) an den Anschlag (32) anstößt, in der Klemmrichtung (K) betrachtet zwischen der Halterung (28) und dem Okularfenster (26) ein Klemmspalt (34) vorhanden ist, wobei das elastische Element (30) zumindest abschnittsweise in dem Klemmspalt (34) angeordnet ist.

6. Okulareinrichtung (20) nach Anspruch 1, **dadurch gekennzeichnet, dass** das elastische Element (30) eine an der Halterung (28), insbesondere an einer Unterseite des Kragens (38) der Halterung (28), einseitig angeschlagene Tellerfeder umfasst, wobei insbesondere vorgesehen ist, dass sich die Tellerfeder ausgehend von einem Anschlagbereich in Richtung ihres freien Endes von einer Außenseite der Halterung (28) in Richtung eines Zentrums der Halterung (28) erstreckt.

7. Okulareinrichtung (20) nach einem der Ansprüche 1 bis 6, **dadurch gekennzeichnet, dass** der Kragen (38) zumindest abschnittsweise als Federelement ausgebildet ist.

8. Okulareinrichtung (20) nach einem der Ansprüche 1 bis 7, **dadurch gekennzeichnet, dass** die Halterung (28) eine Überwurfmutter ist, wobei insbesondere vorgesehen ist, dass die Überwurfmutter ein Innengewinde aufweist, welches im montierten Zustand mit einem an der Okularfensterfassung (22) vorhandenen Außengewinde kämmt.

9. Chirurgisches Instrument (2), insbesondere Endoskop, mit einer Okulareinrichtung (20) nach einem der Ansprüche 1 bis 8.

## Claims

1. An ocular device (20) for a surgical instrument (2), in particular an endoscope, with an optical assembly (24) which can be seated or is seated in an ocular window frame (22) in an interior space of the surgical instrument (2), wherein the optical assembly (24) is separated from an exterior space by an ocular window (26), and the ocular window (26) is seated in a holder (28) which is connectible or connected to the ocular window frame (22), wherein in a connected state, the holder (28) exerts a clamping force on the ocular window (26) in a clamping direction (K), wherein at least one elastic element (30) transmitting the clamping force is comprised, and wherein the holder (28) comprises an edge (36) that is connectible or connected to the ocular window frame (22) and a collar (38) adjacent thereto, wherein the collar (38) surrounds an opening in which the ocular window (26) is at least partially seated, and the collar (38) comprises a first clamping region (40) on its bottom side facing the ocular window (26), wherein the ocular window (26) has a cutout (42) running along its outer perimeter, wherein the ocular window (26) has a second clamping region (44) in the region of the cutout (42) at a top side facing the collar (38), wherein the elastic element (30) is arranged between the first and second clamping region (40, 44), and wherein there is a clamping gap between the first and the second clamping region (40, 44) and the elastic element (30) is arranged in the clamping gap and wherein the ocular window frame (22) has a shoulder (46) that extends along the inner space enclosed by the ocular window frame (22) along the perimeter, **characterized in that** the ocular window (26) is glued to the ocular window frame (22) on an annular surface of the shoulder (46) facing the ocular window (26).

2. The ocular device (20) according to claim 1, **characterized in that** the elastic element (30), viewed in the direction of clamping (K), is at least sectionally arranged between the holder (28) and the ocular window (26).

3. The ocular device (20) according to claim 1 or 2, **characterized in that** the elastic element (30) is a spring element, wherein the spring element is in particular a disc spring, a spring ring, and/or a wave washer.

4. The ocular device (20) according to one of claims 1 to 3, **characterized in that** the ocular window frame (22) comprises a stop (32) for the holder (28) which limits a movement of the holder (28) in the clamping direction (K).

5. The ocular device (20) according to claim 4, **characterized in that** the stop (32) limits a movement of the holder (28) in the clamping direction (K) so that when the holder (28) is in a fastened state when the holder (28) contacts the stop (32), a clamping gap (34) exists between the holder (28) and the ocular window (26) viewed in the direction of clamping (K), wherein the elastic element (34) is arranged at least sectionally in the clamping gap (34).

6. The ocular device (20) according to claim 1, **characterized in that** the elastic element (30) comprises a disc spring contacting one side of the holder (28), in particular the bottom side of the collar (38) of the holder (28), wherein in particular, the disc spring extends from an outer side of the holder (28) toward a center of holder (28) starting from a contact region toward its free end.

7. The ocular device (20) according to one of claims 1 to 6, **characterized in that** the collar (38) is formed at least sectionally as a spring element.

8. The ocular device (20) according to one of claims 1 to 7, **characterized in that** the holder is a union nut (28), wherein in particular the union nut has an inner thread which, in an installed state, meshes with an outer thread on the ocular window frame (22).

9. A surgical instrument (2), in particular an endoscope, with a ocular device (20) according to one of claims 1 to 8.

## Revendications

1. Dispositif d'oculaire (20) pour un instrument chirurgical (2), en particulier un endoscope, avec un ensemble optique (24) qui est reçu ou apte à être reçu dans un support (22) de verre d'oculaire à l'intérieur de l'instrument chirurgical (2), l'ensemble optique (24) étant séparé d'un espace extérieur par un verre d'oculaire (26) et le verre d'oculaire (26) étant reçu dans un support (28) qui est connecté ou apte à être connecté au support (22) de verre d'oculaire, le support (28), à l'état de liaison, exerçant une force de serrage sur le verre d'oculaire (26) dans une direction de serrage (K), au moins un élément élastique (30) étant prévu pour transmettre la force de serrage, et le support (28) ayant un bord (36) qui est connecté ou apte à être connecté au support (22) de verre d'oculaire et une collerette attenante (38), la collerette (38) délimitant une ouverture dans laquelle le verre d'oculaire (26) est au moins partiellement reçu et la collerette (38) comprenant une première zone de serrage (40) sur la face inférieure tournée vers le verre d'oculaire (26), le verre d'oculaire (26) ayant un évidement (42) s'étendant le long de sa circonférence extérieure, le verre d'oculaire (26) présentant une deuxième zone de serrage (44) dans la zone de l'évidement (42), sur une face supérieure tournée vers la collerette (38), une deuxième zone de serrage (44), l'élément élastique (30) étant agencé entre la première et la deuxième zone de serrage (40, 44), et un espace de serrage étant présent entre les première et deuxième zones de serrage (40, 44) et l'élément élastique (30) étant disposé dans l'espace de serrage, et le support (22) de verre d'oculaire présentant un épaulement (46) qui s'étend le long d'un espace intérieur fermé par le support (22) de verre d'oculaire, **caractérisé en ce que** le verre d'oculaire (26) est relié au support (22) de verre d'oculaire sur une surface annulaire de l'épaulement (46) faisant face au verre d'oculaire (26).

2. Dispositif d'oculaire (20) selon la revendication 1, **caractérisé en ce que** l'élément élastique (30), considéré dans le sens de serrage (K), est disposé au moins par endroits entre le support (28) et le verre d'oculaire (26).

3. Dispositif d'oculaire (20) selon la revendication 1 ou la revendication 2, **caractérisé en ce que** l'élément élastique (30) est un élément ressort, l'élément ressort étant notamment une rondelle Belleville, une bague ressort et / ou une rondelle ondulée.

4. Dispositif d'oculaire (20) selon l'une quelconque des revendications 1 à 3, **caractérisé en ce que** le support (22) de verre d'oculaire comprend une butée (32) pour le support (28), ce qui limite le mouvement du support (28) dans le sens de serrage (K).

5. Dispositif d'oculaire (20) selon la revendication 4, **caractérisé en ce que** la butée (32) limite le mouvement du support (28) dans le sens de serrage (K) de telle sorte qu'à l'état de serrage du support (28), lorsque le support (28) est en butée contre la butée (32), vu dans le sens de serrage (K), un espace de serrage (34) existe entre le support (28) et le verre d'oculaire (26), l'élément élastique (30) étant agencé au moins par endroits dans l'espace de serrage (34).

6. Dispositif d'oculaire (20) selon la revendication 1, **caractérisé en ce que** l'élément élastique (30) comprend une rondelle Belleville montée d'un côté sur le support (28), en particulier sur une face inférieure de la collerette (38) du support (28), la rondelle Belleville s'étendant notamment à partir d'une zone de butée dans la direction de son extrémité libre depuis un côté extérieur du support (28) en direction d'un centre du support (28).

7. Dispositif d'oculaire (20) selon l'une des revendications 1 à 6, **caractérisé en ce que** la collerette (38) est conçue au moins par endroits sous la forme d'un élément ressort.

8. Dispositif d'oculaire (20) selon l'une quelconque des revendications 1 à 7, **caractérisé en ce que** le support (28) est un écrou-raccord, l'écrou-raccord présentant notamment un filetage intérieur, qui, à l'état d'assemblage, vient en prise avec un filetage extérieur présent sur le support (22) de verre d'oculaire.

9. Instrument chirurgical (2), notamment endoscope, ayant un dispositif d'oculaire (20) selon l'une des revendications 1 à 8.
